# EUROPÄISCHE PATENTSCHRIFT

(11) **EP 3 727 131 B1**
(45) Veröffentlichungstag und Bekanntmachung des Hinweises auf die Patenterteilung: **13.08.2025**
(21) Anmeldenummer: 18827040.9
(22) Anmeldetag: 18.12.2018
(51) Int. Cl.: A61B 5/00, A61B 5/0215, A61F 2/86

(54) **PASSIVES TRANSPONDERSYSTEM UND DRUCKWELLENMESSVORRICHTUNG**
PASSIVE TRANSPONDER SYSTEM AND PRESSURE WAVE MEASURING DEVICE
SYSTÈME DE TRANSPONDEUR PASSIF ET DISPOSITIF DE MESURE D'ONDE DE PRESSION

(30) Priorität: 22.12.2017 DE 102017223695
(43) Veröffentlichungstag der Anmeldung: 28.10.2020
(73) Patentinhaber: VesselSens GmbH, 53175 Bonn (DE)
(72) Erfinder: DOMNICH, Alexej, 53229 Bonn (DE); TUTSCH, Fabian, 53175 Bonn (DE)
(74) Vertreter: Pfenning, Meinig & Partner mbB
(86) Internationale Anmeldenummer: PCT/EP2018/085523
(87) Internationale Veröffentlichungsnummer: WO 2019/121700

(56) Entgegenhaltungen:
- WO-A1-02/098296
- WO-A1-2006/096685
- WO-A1-2012/100959
- WO-A2-2007/078770
- US-A1- 2005 080 346
- AKAR O ET AL: "A wireless batch sealed absolute capacitive pressure sensor", SENSORS AND ACTUATORS A: PHYSICAL, ELSEVIER BV, NL, vol. 95, no. 1, 15 December 2001 (2001-12-15), pages 29 - 38, XP004328628, ISSN: 0924-4247, DOI: 10.1016/S0924-4247(01)00753-1
- ZURICH INSTRUMENTS: "Principles of lock-in detection and the state of the art", 1 November 2016 (2016-11-01), XP055567389, Retrieved from the Internet <URL:https://www.zhinst.com/sites/default/files/li_primer/zi_whitepaper_principles_of_lock-in_detection.pdf> [retrieved on 20190311]

## Beschreibung

Die Erfindung betrifft ein passives Transpondersystem mit einer ersten oder zweiten Leiterschleifenstruktur sowie einem ersten und einem zweiten kapazitiven Drucksensor, wobei jeweils eine Leiterschleifenstruktur mit einem der kapazitiven Drucksensoren zu einem Schwingkreis gekoppelt ist, wobei die erste Leiterschleifenstruktur zur zweiten Leiterschleifenstruktur in einem nicht verschwindenden Winkel steht. Die Resonanzfrequenzen der Schwingkreise sind dabei so gewählt, dass sie sich nicht schwebend überlagern. Die Erfindung betrifft außerdem eine Druckwellenmessvorrichtung mit einem solchen passiven Transpondersystem sowie einer Ausleseeinheit.

Für verschiedene Anwendungen werden Vorrichtungen eingesetzt, die eine induktiv gekoppelte Kommunikation zwischen einem Transponder oder einem passiven Resonanzkreis und einem Lesegerät einsetzen. Hierbei wird der Transponder bzw. der passive Resonanzkreis mittels induktiver Kopplung mit Energie versorgt. Gleichzeitig erfolgt über eine solche Kopplung eine Signalübertragung und damit Informationsübertragung.

Derartige Vorrichtungen werden zum Beispiel in der Medizin eingesetzt, um Informationen über den Blutfluss in Gefäßen zu erhalten. Der Transponder kann hierbei als Spule ausgestaltet sein, die das betreffende Blutgefäß umläuft.

Problematisch ist, dass solche Spulen eine ausgeprägte achtförmige Richtcharakteristik aufweisen. Dies führt dazu, dass die Kopplung zwischen der Auslesespule und der Transponderspule entscheidend von der Orientierung der Transponderspule gegenüber der Auslesespule abhängt. Bei maximaler Durchdringung der Transponderspule von dem durch die Auslesespule erzeugten Feld kann ein maximales Signal erzielt werden. Wird hingegen die Sensorspule überhaupt nicht vom Feld der Auslesespule durchdrungen, z. B. wenn diese in einem Winkel von 90° zueinander stehen, wird keinerlei Kopplung erreicht und das Auslesen wird unmöglich.

Eine mögliche Ausgestaltung der Transponderspule ist eine sattelförmige Wicklung, bei der die Leiterbahnen der Spule einer Zylinderfläche folgen und die Spulenachse senkrecht zur Zylinderachse dieser Zylinderfläche steht.

Wird die Transponderspule implantiert, so ist nicht oder nur sehr schwer bestimmbar, wie solche Sattelspule um das entsprechende Blutgefäß orientiert ist. Steht die Achse der Sattelspule parallel zur Achse der Auslesespule, so ist die Kopplung maximal. Stehen die Spulenachsen senkrecht zueinander, so wird keinerlei Kopplung erzielt.

Die WO02/098296 A1 beschreibt ein endovaskuläres Implantat oder Endotransplantat umfassend eine röhrenförmige Hülse mit integralen Innen- und Außenschichten. Zwischen den beiden Schichten ist ein Drucksensor eingebettet und von diesen abgedeckt. Und die Hülse ist am Drucksensor flexibel, um eine Druckübertragung durch die Hülse zur Erkennung durch den verwendeten Drucksensor zu ermöglichen.

Die WO2012/100959 A1 betrifft eine zylinderförmige Vorrichtung zum Durchfluss von Fluid, die zumindest zwei Drucksensoren aufweist, mit welchen ein Druck des Fluids im Inneren der Vorrichtung messbar ist.

Die WO 2006/096685 A1 zeigt eine Hochfrequenzantennenanordnung für ein medizinisches Gerät bereitgestellt, beispielsweise für ein Gerät, das in einen Patienten implantiert werden kann. Die Antennenbaugruppe umfasst mehrere Antennen, die jeweils darauf ausgerichtet sind, ein Hochfrequenzsignal zu empfangen, das sich in einer anderen Richtung ausbreitet. Die einzelnen elektrischen Signale, die in jeder Antenne erzeugt werden, werden additiv zu einem einzigen Signal kombiniert, das eine größere Stärke als jedes der einzelnen elektrischen Signale hat.

Aufgabe der vorliegenden Erfindung ist es, einen passiven Transponder anzugeben, der zuverlässig und kontrollierbar eine gute Kopplung mit einer Auslesespule ermöglicht. Aufgabe ist es außerdem, eine Druckwellenmessvorrichtung anzugeben, in der eine gute und kontrollierbare Auslesung des Signals ermöglicht wird.

Diese Aufgabe wird gelöst durch das passive Transpondersystem nach Anspruch 1 und die Druckwellenmessvorrichtung nach Anspruch 10. Die jeweiligen abhängigen Ansprüche geben vorteilhafte Weiterbildungen des passiven Transponderssystems nach Anspruch 1 und der Druckwellenmessvorrichtung nach Anspruch 10 an.

Erfindungsgemäß wird ein passives Transpondersystem angegeben, das eine erste Leiterschleifenstruktur mit zumindest einer Windung sowie eine zweite Leiterschleifenstruktur mit zumindest einer Windung aufweist. Unter einer Leiterschleifenstruktur kann hierbei eine aus einem elektrischen Leiter geschaffene Struktur verstanden werden, die zumindest eine Windung des Leiters aufweist. Der Leiter ist hierbei vorzugsweise selbst länglich ausgestaltet, beispielsweise als Draht oder als Leiterbahn, und entlang seiner Längsrichtung zur zumindest einen Windung geformt.

Das passive Transpondersystem weist außerdem einen ersten kapazitiven Drucksensor und einen zweiten kapazitiven Drucksensor auf. Unter einem kapazitiven Drucksensor wird hierbei eine Kapazität, also ein Kondensator, verstanden, deren Wert von einem Druck abhängt, mit dem der Sensor beaufschlagt wird. In einem einfachen Beispiel kann ein solcher kapazitiver Drucksensor als abgeschlossenes Volumen ausgestaltet sein, auf dessen gegenüberliegenden Oberflächen jeweils eine Kondensatorplatte angeordnet ist. Wird eine solche Struktur mit einem Druck beaufschlagt, so verändert sich der Abstand zwischen den Kondensatorplatten und damit die Kapazität des durch die Kondensatorplatten gebildeten Kondensators. Die Kapazität kann daher als Maß für den beaufschlagten Druck verwendet werden.

Erfindungsgemäß ist der erste kapazitive Drucksensor mit der ersten Leiterschleifenstruktur zu einem ersten Schwingkreis gekoppelt. Dieser erste Schwingkreis hat eine Resonanzfrequenz, die im Folgenden als "erste Resonanzfrequenz" bezeichnet werden soll. Darüber hinaus ist der zweite kapazitive Drucksensor mit der zweiten Leiterschleifenstruktur zu einem zweiten Schwingkreis elektrisch gekoppelt, dessen Resonanzfrequenz als "zweite Resonanzfrequenz" bezeichnet werden soll.

Die Windungen der Leiterschleifenstrukturen sind jeweils um zumindest eine Windungsachse gewunden. Es ist also die zumindest eine Windung der ersten Leiterschleifenstruktur und zumindest eine erste Windungsachse gewunden und die zumindest eine Windung der zweiten Leiterschleifenstruktur um zumindest eine zweite Windungsachse. Als Windungsachse soll hierbei eine Gerade verstanden werden, die senkrecht auf einer Fläche steht, in der die entsprechende Windung verläuft. Vorzugsweise soll die Windungsachse senkrecht auf einem Mittelpunkt des von der entsprechenden Windung umschlossenen Bereichs dieser Fläche stehen. Als Mittelpunkt kann hierbei jener Punkt angesehen werden, gegenüber dem die entsprechende Windung punktsymmetrisch ist. Dass die Windung punktsymmetrisch ist, ist hierbei optional. Die Punktsymmetrie liegt vorzugsweise in Projektion auf eine Ebene vor, auf der die Windungsachse senkrecht steht. Die Windungsachse durchtritt diese Ebene vorzugsweise in einem Mittelpunkt der Projektion der Windung auf diese Ebene. Eine schneckenhausförmige Windung, also eine Windung, die nach Umlauf um die Windungsachse einen etwas größeren oder kleineren Radius hat als an ihrem gegenüberliegenden Ende soll hier ebenfalls als punktsymmetrisch in diesem Sinne angesehen werden.

Erfindungsgemäß stehen die zumindest eine erste Windungsachse und die zumindest eine zweite Windungsachse in einem nicht verschwindenden Winkel zueinander. Der Winkel ist also größer als Null und kleiner als 180°. Der Winkel soll nicht 0° und nicht 180° sein. Bevorzugterweise ist der Winkel 90°. Die Windungsachsen müssen sich erfindungsgemäß nicht schneiden. In diesem Fall wird der Winkel zwischen Projektionen der Windungsachsen in Richtung senkrecht zu einer oder beider der Windungsachsen auf einer gemeinsamen Ebene gemessen.

Das passive Transpondersystem der Erfindung kann auch als passiver Transponder bezeichnet werden. Der erste Schwingkreis und der zweite Schwingkreis können vorteilhafterweise strukturell miteinander verbunden sein, es ist aber auch möglich, die beiden Schwingkreise nicht miteinander strukturell verbunden zu realisieren.

Bekannterweise kann die Resonanzfrequenz eines Schwingkreises durch die Wahl der Kapazität und der Induktivität der Leiterschleifenstruktur eingestellt werden. Vorzugsweise sind die erste Resonanzfrequenz und die zweite Resonanzfrequenz zumindest soweit unterschiedlich, dass sie sich nicht schwebend überlagern. Die erste Resonanzfrequenz und die zweite Resonanzfrequenz sind also vorzugsweise so gewählt, dass, wenn sie überlagert werden, sie keine Schwebung bilden. Hierdurch lassen sich die von den einzelnen Schwingkreisen erzeugten Signale besonders gut trennen.

Als vorteilhaft hat es sich erwiesen, wenn die Resonanzfrequenz des ersten und zweiten Schwingkreises sich um zumindest die zweifache Bandbreite des ersten oder des zweiten Schwingkreises unterscheiden. Vorteilhafterweise unterscheiden sich die Resonanzfrequenzen des ersten und des zweiten Schwingkreises zumindest um die zweifache Bandbreite jenes dieser beiden Schwingkreise, der die größere Bandbreite hat. Vorteilhafterweise ist die Bandbreite dabei die sog. 3 dB Bandbreite, welche die Differenz der oberen -3 dB Grenzfrequenz f₂ und der unteren -3dB Grenzfrequenz f₁ ist. Die Grenzfrequenzen sind dabei jene Frequenzen, bei denen die Resonanzkurve jeweils den Wert -3dB schneidet. Die Bandbreite B kann auch definiert werden als B = f₂ - f₁ = f₀/Q, wobei f₀ = √(f₁f₂) ist und Q die Güte des Schwingkreises. Bei gängigen Sensorschwingkreisen könnte der Mindestabstand also beispielsweise bei 2 MHZ liegen.

In einer vorteilhaften Ausgestaltung können die erste und die zweite Leiterschleifenstruktur als Flachspulen ausgebildet sein, die auf Oberflächen einer Trägerstruktur angeordnet sind. Weisen die Leiterschleifenstrukturen hierbei mehr als eine Windung auf, so können diese Windungen jeweils auf einer gemeinsamen Fläche verlaufen, wobei benachbarte der Windungen jeweils einen etwas größeren Durchmesser haben, so dass sich benachbarte Windungen umschließen. Auf diese Weise können Spulen erzeugt werden, die flach sind und beliebig viele Windungen haben. In dieser Ausführungsform kann die Fläche der Flachspulen, d. h. jene Fläche, in der die Windungen verlaufen, die Oberfläche der Trägerstruktur sein.

Besonders vorteilhaft kann die erste Leiterschleifenstruktur dabei auf einer innenseitigen Oberfläche der Trägerstruktur angeordnet sein und die zweite Leiterschleifenstruktur auf einer außenseitigen Oberfläche. Es ist aber auch möglich, dass beide Leiterschleifenstrukturen auf der gleichen Oberfläche der Trägerstruktur angeordnet sind, wobei dann vorteilhaft eine Isolierung der Leiterschleifenstrukturen gegeneinander vorgesehen wird. Hierzu können beispielsweise die Leiterbahnen der Leiterschleifenstrukturen mit einem Isolator ummantelt sein. Möglich ist es auch, zunächst eine der Leiterschleifenstrukturen aufzubringen, dann diese gesamte Oberfläche der Trägerstruktur mit einem isolierenden Material zu beschichten und dann die andere Leiterschleifenstruktur aufzubringen. Auf diese Weise können beliebig viele Leiterschleifenstrukturen auf der gleichen Oberfläche der Trägerstruktur angeordnet werden. Es ist aber auch möglich, dass die Leiterbahn einer der Leiterschleifenstrukturen sich nur dort zur anderen Oberfläche der Trägerstruktur erstreckt, wo sie eine Leiterbahn der anderen Leiterschleifenstruktur kreuzt.

Vorteilhafterweise kann die Trägerstruktur die Form eines Teilzylinders, eines Zylinders, eines Teilschlauches, eines Schlauches, eines Teilrohres oder eines Rohres haben. Unter einem Zylinder kann hier eine Fläche verstanden werden, die an allen Punkten denselben Abstand von einer gemeinsamen Linie hat. Diese Linie soll hier als Zylinderachse bezeichnet werden. Dabei kann die Linie gekrümmt sein oder eine Gerade sein. Vorzugsweise ist die Fläche um die genannte Linie herum geschlossen. Besonders bevorzugt ist eine Grundfläche des Zylinders kreisförmig, andere Formen der Grundfläche sind aber ebenfalls möglich. Ein Zylinder mit einer kreisförmigen Grundfläche kann auch als Schlauch oder als Rohr bezeichnet werden, wobei im Falle eines Schlauches die besagte Linie gekrümmt sein kann, und wobei im Falle eines Rohres die genannte Linie eine Gerade sein kann. Unter einem Teilzylinder kann hier eine Form verstanden werden, deren Punkte vollständig Teil einer Zylinderfläche sind, die aber keine vollständige Zylinderfläche bildet. Entsprechend kann unter einem Teilschlauch oder Teilrohr eine Fläche verstanden werden, deren Punkte vollständig Teil eines Schlauches oder Rohres sind, dieses aber nicht vollständig ausfüllt. Die Trägerstruktur kann dann zum Beispiel die Form eines zu drei Vierteln geschlossenen Schlauches oder Rohres haben. Für eine Verwendung in Blutgefäßen sind die Schlauchform und die Rohrform bevorzugt, da sie der Geometrie des Blutgefäßes angepasst sind.

In dieser Ausgestaltung kann der Winkel zwischen den Windungsachsen der ersten und der zweiten Leiterschleifenstruktur im Rahmen des von der Erfindung Vorgegebenen frei gewählt werden. Sind die Leiterschleifenstrukturen auf Oberflächen einer rohrförmigen oder schlauchförmigen Struktur angeordnet, so können sie sattelförmig ausgestaltet sein. Besonders vorteilhaft können sie dabei so ausgestaltet sein, dass die Leiterschleifen bzw. Windungen jeweils zwei gerade Abschnitte aufweisen, in denen sie parallel zu einer Zylinderachse der Trägerstruktur verlaufen, wobei die geraden Abschnitte jeweils durch kreisbogenförmige Abschnitte miteinander verbunden sind, in denen die entsprechende Windung kreisbogenförmig um die Zylinderachse der Trägerstruktur verläuft. Die Drucksensoren können hierbei ebenfalls auf den Oberflächen der Trägerstruktur angeordnet sein, wobei vorzugsweise der erste kapazitive Drucksensor auf jener Oberfläche der Trägerstruktur angeordnet ist, auf der auch die erste Leiterschleifenstruktur angeordnet ist und der zweite kapazitive Drucksensor auf jener Oberfläche der Trägerstruktur angeordnet ist, auf der auch die zweite Leiterschleifenstruktur angeordnet ist.

Die kreisbogenförmigen Abschnitte der Windungen haben dabei vorzugsweise eine Länge von 180°, was gleichbedeutend ist mit der Aussage, dass die jeweils zwei geraden Abschnitte der Windungen sich bezüglich der Zylinderachse genau gegenüberliegen. Hierdurch wird die von der Windung umlaufene Fläche maximal und dadurch die Kopplung maximiert.

Als Trägerstruktur kommt besonders vorteilhaft ein Kunststoffrohr oder ein Kunststoffschlauch in Frage. Die beiden Leiterschleifenstrukturen werden dann durch das Kunststoffrohr oder den Kunststoffschlauch elektrisch gegeneinander isoliert. Die Trägerstruktur kann beispielsweise aus Polyimid hergestellt sein.

Vorzugsweise stehen die Spulenachsen senkrecht aufeinander im oben beschriebenen Sinne, wodurch die gegenseitige Beeinflussung minimiert wird und eine der Kreisform am meisten angepasste Richtcharakteristik erzielt wird. Bei Verwendung kann die Zylinderachse der Trägerstruktur koaxial liegen zur Längsachse eines Blutgefäßes an dem die Trägerstruktur angeordnet ist. Da die Blutgefäße im menschlichen Körper aufgrund der Anatomie zum größten Teil parallel zur längsten Körperausdehnung verlaufen, wird die genannte Richtcharakteristik vorzugsweise in einer Ebene der Kreisform angenähert, die senkrecht zur Zylinderachse oder Längsachse der Trägerstruktur liegt. Die Richtungsabhängigkeit ist insbesondere deshalb in dieser radialen Richtung vorteilhaft, weil eine Korrektur durch Verschieben der Auslesespule um das Gefäß herum nur dann ohne Signaleinbußen möglich wäre, wenn sich das Gefäß exakt in der Mitte des zylindrischen Körperteils befindet, was de facto nur in wenigen Ausnahmefällen der Fall ist. In allen anderen Fällen wird sich das Gefäß in einer Richtung näher an der Oberfläche des Körperteils befinden und genau an dieser Stelle sollte die Kopplung mit der Transponderspule auch möglichst gut sein. Eine axiale Verschiebung der Auslesespule zur Sensorspule ist da zwar möglich, kann aber durch einfaches Verschieben der Auslesespule entlang des Körperteils korrigiert werden.

Die genannte Flachspule kann vorteilhaft einen runden, ovalen, quadratischen oder rechteckigen Umfang haben, wobei die entsprechenden Formen jeweils mit der entsprechenden Oberfläche der Trägerstruktur gekrümmt sind. Vorzugsweise ist die mittlere Breite der Spule im ungebogenen, also flachen, Zustand gleich dem Dreiviertelstel des Umfangs der Trägerstruktur.

Erfindungsgemäß weist die erste Leiterschleifenstruktur eine Mehrzahl von in Richtung einer Hauptachse nebeneinander angeordneten Gruppen von Windungen auf. Jede Gruppe weist dabei zumindest eine Windung auf. Auch eine einzelne Windung kann daher als Gruppe in diesem Sinne verstanden werden.

Dabei ist jede der Gruppen von Windungen der ersten Leiterschleifenstruktur um eine eigene erste Windungsachse gewunden, wobei vorzugsweise die ersten Windungsachsen unterschiedlicher der Gruppen zueinander parallel stehen und voneinander um einen nicht verschwindenden Abstand beabstandet sind. Vorzugsweise sind diese Abstände für alle jeweils benachbarten Gruppen von Windungen der ersten Schleifenstrukturen gleich. Windungen der gleichen Gruppe von Windungen sind um die gleiche Windungsachse gewunden. Die Windungsachsen von unterschiedlichen der Gruppen sind unterschiedlich, also nicht koaxial, und voneinander beabstandet.

Entsprechend weist die zweite Leiterschleifenstruktur eine Mehrzahl an in Richtung der Hauptachse nebeneinander angeordneten Gruppen von Windungen auf. Jede Gruppe weist dabei zumindest eine Windung auf. Auch eine einzelne Windung kann daher als Gruppe in diesem Sinne verstanden werden. Es ist dann wiederum jede der Gruppen von Windungen der zweiten Leiterschleifenstruktur um eigene zweite Windungsachsen gewunden, wobei die zweiten Windungsachsen vorzugsweise zueinander parallel stehen und voneinander um einen nicht verschwindenden Abstand beabstandet sind. Auch hier sind vorzugsweise diese Abstände für alle jeweils benachbarten zweiten Windungsachsen gleich groß. Windungen der gleichen Gruppe von Windungen sind wiederrum um die gleiche Windungsachse gewunden. Die Windungsachsen von unterschiedlichen der Gruppen können unterschiedlich, also nicht koaxial, und voneinander beabstandet sein.

Dass Windungen nebeneinander angeordnet sind, bedeutet dabei zunächst, dass die Windungsachsen nebeneinander angeordnet sind. Windungen benachbarter Gruppen der gleichen Leiterschleifenstruktur, die nebeneinander angeordnet sind, können einander überlappen, unmittelbar aneinandergrenzen oder voneinander beabstandet sein.

Weist jede Gruppe nur eine Windung auf, so kann die erste Leiterschleifenstruktur eine Mehrzahl an in Richtung einer Hauptachse nebeneinander angeordneten Windungen aufweisen, wobei jeder der Windungen der ersten Leiterschleifenstruktur um eine eigene Windungsachse gewunden ist, wobei die ersten Windungsachsen voneinander um einen nicht verschwindenden Abstand beabstandet sind. Entsprechend kann die zweite Leiterschleifenstruktur einen Mehrzahl an in Richtung der Hauptachse nebeneinander angeordnete Windungen aufweisen, wobei jede der Windungen der zweiten Leiterschleifenstruktur um eine eigene zweite Windungsachse gewunden ist, wobei die zweiten Windungsachsen einen nicht verschwindenden Abstand voneinander haben.

Bevorzugterweise liegen die Windungsachsen von nebeneinander angeordneten der Windungen oder von Gruppen von Windungen der gleichem Leiterschleifenstruktur parallel zueinander.

Besonders bevorzugt sind außerdem die Abstände, um welche die zweiten Windungsachsen voneinander beabstandet sind, gleich groß zu jenen Abständen, um die die ersten Windungsachsen voneinander beabstandet sind. Durch diese Ausgestaltung lässt sich eine der Kreisform bestmöglich angenäherte Richtcharakteristik in einer Ebene senkrecht zu jener Richtung erzielen, in der die Windungsachsen nebeneinander angeordnet sind. Außerdem kann über die gesamte Länge in Richtung der Nebeneinanderanordnung der Windungsachsen eine homogene Richtcharakteristik erzielt werden. Eine solche Leiterschleifenstruktur kann deutlich länger sein, als die Durchmesser.

In dieser Ausgestaltung der Erfindung können die erste und die zweite Leiterschleifenstruktur jeweils mittels eines Drahtes hergestellt sein. Besonders vorteilhaft kann dabei ein Draht, der die erste Leiterschleifenstruktur bildet, durch Windungen jenes Drahtes geführt sein, der die zweite Leiterschleifenstruktur bildet. Auf diese Weise sind die erste und die zweite Leiterschleifenstruktur vorteilhaft miteinander verwoben und können so eine stabile Struktur bilden. Eine solche kann beispielsweise als Stent für ein Blutgefäß dienen. In entsprechender Weise kann auch der die zweite Leiterschleifenstruktur bildende Draht durch die Windungen der ersten Leiterschleifenstruktur geführt sein.

In einer vorteilhaften Ausgestaltung der Erfindung können sich die Windungen der ersten Leiterschleifenstruktur in zwei bezüglich der Hauptachse gegenüberliegenden Flächen erstrecken, wobei vorteilhafterweise die ersten Windungsachsen jeweils senkrecht auf diesen gegenüberliegenden Flächen stehen. Vorteilhafterweise können sich auch die Windungen der zweiten Leiterschleifenstruktur in zwei bezüglich der Hauptachse gegenüberliegenden Flächen erstrecken, auf denen dann die zweiten Windungsachsen jeweils senkrecht stehen. Dass sich die zwei Flächen bezüglich der Hauptachse gegenüberliegen bedeutet hierbei, dass diese Flächen beide senkrecht auf einer Geraden stehen, die die Hauptachse schneidet, wobei die Hauptachse zwischen diesen beiden gegenüberliegenden Flächen angeordnet ist. Diese Ausgestaltung kann sowohl in der vorstehend beschriebenen sattelförmigen Ausgestaltung der Leiterschleifenstrukturen, als auch in der Ausgestaltung mit nebeneinander angeordneten Windungen vorteilhaft zum Einsatz kommen.

Weisen die erste und die zweite Leiterschleifenstruktur eine Mehrzahl an in Richtung der Hauptachse nebeneinander angeordneten Windungen auf, so können sich diese Windungen in entsprechender Weise in zwei bezüglich der Hauptachse gegenüberliegenden Flächen erstrecken. Dabei können besonders vorteilhaft jeweils die Windungen der ersten Leiterschleifenstruktur durch die Windungen der zweiten Leiterschleifenstruktur hindurch verlaufen, so dass die erste und die zweite Leiterschleifenstruktur ineinander verwoben sind. Entlang des Umfangs kann dann jeweils eine Windung der ersten Leiterschleifenstruktur durch eine Windung der zweiten Leiterschleifenstruktur verlaufen, wobei diese Windung der zweiten Leiterschleifenstruktur auf ihrer der besagten ersten Windung abgewandten Seite durch eine Windung der ersten Leiterschleifenstruktur verläuft. Diese zuletzt genannte Windung der ersten Leiterschleifenstruktur kann dabei auf jener Fläche verlaufen, die der Fläche gegenüberliegt, in der die zuerst genannte Windung der ersten Leiterschleifenstruktur verläuft. Die zuletzt genannte Windung der ersten Leiterschleifenstruktur kann nun mit einer weiteren Windung der zweiten Leiterschleifenstruktur ineinander greifen, welche ihrerseits mit der zuerst genannten Windung der ersten Leiterschleifenstruktur ineinander greift. Auf diese Weise kann mit den Windungen der ersten und zweiten Leiterschleifenstruktur ein die Hauptachse vollständig umgebendes Gewebe gebildet werden, in dem sich jeweils Windungen der ersten Leiterschleifenstruktur gegenüberliegen und die Windungen der zweiten Leiterschleifenstruktur gegenüberliegen. Die Windungen der ersten Leiterschleifenstruktur und der zweiten Leiterschleifenstruktur greifen dabei jeweils ineinander. In einer vorteilhaften Ausgestaltung der Erfindung können die ersten Windungsachsen der ersten Leiterschleifenstruktur in Richtung entlang der Hauptachse zwischen den zweiten Windungsachsen der zweiten Leiterschleifenstruktur liegen. Auf diese Weise kann erreicht werden, dass ein von den Windungen der ersten Leiterschleifenstruktur erzeugtes Magnetfeld die Windungen der zweiten Leiterschleifenstruktur möglichst wenig durchsetzt und ein von den Windungen der zweiten Leiterschleifenstruktur erzeugtes Magnetfeld die Windungen der ersten Leiterschleifenstruktur möglichst wenig durchsetzt. Dies verbessert die Richtcharakteristik in radialer Richtung.

Damit die ersten und zweiten Leiterschleifenstrukturen wie vorbeschrieben ineinander verwoben werden können, ist es vorteilhaft, wenn der Draht, aus dem die erste und die zweite Leiterschleifenstruktur gebildet sind, mit einer elektrischen Isolierung ummantelt sind. Verwendet werden kann beispielsweise DFT-Draht, der einen Kern aus Au oder Ag aufweist und mit NiTi ummantelt ist.

Die beschriebene Verflechtung der ersten und zweiten Leiterschleifenstruktur entspricht vom elektrischen und elektromagnetischen Verhalten her zwei oder auch mehr im gewünschten Winkel zueinander gegeneinander verdrehten Spulen. Durch die Verflechtung besitzt die Spule Bereiche, die die eigentlichen Spulen darstellen sowie Kreuzungspunkte, an denen diese Spulen mechanisch miteinander zu einem zylindrischen Gesamtgebilde zusammengefasst und gegeneinander fixiert werden können. Eine derartige Ausgestaltung kann gleichermaßen als Spule und als Gefäßstütze, z. B. als Stent, wirken. Sie kann aus Material hergestellt werden, das sowohl elektrische, als auch mechanische sowie biokompatible Eigenschaften haben kann. Dadurch, dass die Spulenachsen in einem nicht verschwindenden Winkel zueinander stehen, kann die beschriebene gute Kopplung unabhängig vom Winkel erzielt werden.

In einer vorteilhaften Ausgestaltung der Erfindung können die kapazitiven Drucksensoren mit ihrer druckmessenden Fläche in einer jener Flächen liegen, in denen sich die Leiterschleifenstruktur des entsprechenden Schwingkreises erstreckt. Es kann also der erste kapazitive Drucksensor vorteilhafterweise mit seiner druckmessenden Oberfläche in einer jener Flächen angeordnet sein, in denen die erste Leiterschleifenstruktur verläuft und es kann vorteilhafterweise der zweite kapazitive Drucksensor mit seiner druckmessenden Fläche in einer jener Flächen angeordnet sein, in denen die zweite Leiterschleifenstruktur verläuft.

Bevorzugterweise können die erste Leiterschleifenstruktur und/oder die zweite Leiterschleifenstruktur jeweils aus einem einzigen Draht geformt sein. Dieser kann dabei zu allen Windungen der entsprechenden Leiterschleifenstruktur gebogen sein. Vorteilhaft können auch in Richtung des Umfangs um die Hauptachse nebeneinanderliegende Windungen der ersten und der zweiten Leiterstruktur anders miteinander verbunden sein, als durch Ineinandergreifen. Beispielsweise können diese Windungen miteinander verklebt sein. Eine Ausgestaltung mit ineinandergreifenden Leiterstrukturen ist jedoch insbesondere vorteilhaft, weil hier für das Verbinden keine weiteren Produktionsschritte erforderlich sind, so dass die Herstellbarkeit einfacher ist.

Vorteilhafterweise kann die erste Resonanzfrequenz größer oder gleich 5 MHz, vorzugsweise größer oder gleich 10 MHz, besonders bevorzugt größer oder gleich 15 MHz sein und/oder kleiner oder gleich 40 MHz, vorzugsweise kleiner oder gleich 30 MHz, besonders bevorzugt kleiner oder gleich 20 MHz.

Erfindungsgemäß wird außerdem eine Druckwellenmessvorrichtung bereitgestellt, die zum einen ein passives Transpondersystem, wie vorstehen beschrieben, aufweist sowie darüberhinaus eine Ausleseeinheit. Die Ausleseeinheit weist dabei zumindest eine Auslesespule auf, die so gegenüber dem passiven Transpondersystem anordenbar ist, dass ein durch sie erzeugtes Magnetfeld zumindest eine der Leiterschleifenstrukturen des Transponderssystems durchsetzt. Die erfindungsgemäße Druckwellenmessvorrichtung weist außerdem eine Auswerteelektronik auf, mit der die Auslesespule mit einem die Schwingkreise des passiven Transpondersystems anregenden Signal beaufschlagbar ist. Als Signal wird hierbei ein elektromagnetisches Wechselfeld verstanden, das die Leiterschleifenstrukturen des passiven Transpondersystems durchsetzen kann, wenn es in geeignetem Abstand und geeigneter Ausrichtung gegenüber dem passiven Transpondersystems angeordnet wird. Mit der Auswerteelektronik kann erfindungsgemäß außerdem ein von der Auslesespule empfangenes Signal ausgewertet werden. Dabei ist das von der Auslesespule empfangene Signal ein zeitlicher Stromverlauf, der dadurch erzeugt wird, dass die Auslesespule von einem elektromagnetischen Feld durchsetzt wird, das durch die Schwingkreise des passiven Transpondersystems erzeugt wird.

In einer vorteilhaften Ausgestaltung der Erfindung kann die Druckwellenmessvorrichtung eine Signalquelle aufweisen, mit der ein Signal, also ein Wechselstromverlauf, mit den Resonanzfrequenzen der Schwingkreise erzeugbar ist. Vorteilhafterweise weist die Druckwellenmessvorrichtung darüberhinaus außerdem einen Richtkoppler auf, an dessen Ausgang die Signalquelle elektrisch gekoppelt ist und an dessen Eingang die Auslesespule gekoppelt ist.

Vorteilhafterweise kann das Signal mit der Form a sin (ωₐt) + b sin (ω_{b}t) haben.

In einer weiteren vorteilhaften Ausgestaltung der Erfindung kann die Druckwellenmessvorrichtung einen ersten Mischer aufweisen, in den ein von der Auslesespule empfangenes Signal einleitbar ist. Hinter dem ersten Mischer kann ein erster Tiefpassfilter vorgesehen sein, in den ein vom Mischer ausgegebenes Signal eingeleitet werden kann. Mit dem ersten Mischer kann ein durch die Auslesespule vom Transpondersystem empfangenes Signal mit der ersten Resonanzfrequenz heruntergemischt werden. Die Druckwellenmessvorrichtung der Erfindung kann außerdem einen weiteren zweiten Mischer aufweisen, in den ein von der Auslesespule empfangenes Signal einleitbar ist. Hinter diesem weiteren Mischer kann ein zweiter Tiefpassfilter vorgesehen sein, in den ein vom zweiten Mischer ausgegebenes Signal einleitbar ist. Es kann wiederum mit dem zweiten Mischer ein durch die Auslesespule vom Transpondersystem empfangenes Signal der zweiten Resonanzfrequenz heruntermischbar sein.

Bevorzugterweise ist die Druckwellenmessvorrichtung so eingerichtet, dass das mit der ersten Resonanzfrequenz empfangene Signal mit dem mit der zweiten Resonanzfrequenz empfangenen Signal konstruktiv addierbar ist. Ist wie oben beschrieben ein Mischer und ein Tiefpassfilter vorgesehen, so ist vorteilhafterweise das vom ersten Tiefpassfilter ausgegebene Signal mit dem vom zweiten Tiefpassfilter ausgegebenen Signal konstruktiv addierbar. Diese konstruktive Addition kann dadurch erfolgen, dass zum einen das vom ersten Tiefpassfilter ausgegebene Signal in Absolutbetrag bzw. Amplitude und Phase unterteilt wird und außerdem das vom zweiten Tiefpassfilter ausgegebene Signal in Amplitude bzw. Absolutbetrag und Phase unterteilt wird. Es können dann die Absolutbeträge bzw. Amplituden miteinander addiert werden und die Phasen miteinander addiert werden, so dass sich eine Summenamplitude und eine Summenphase ergibt.

Das Messprinzip kann einem Lock-in-Verstärker entsprechen. Der erste Schwingkreis und der zweite Schwingkreis liefern eine X-Komponente und eine Y-Komponente, die von der Auslesespule empfangen werden. Sind ωₐ und ω_{b} die Resonanzfrequenzen der beiden Schwingkreise des passiven Transpondersystems bei einem bestimmten Triggerdruck pt, so kann das Signal wie oben beschrieben vorgegeben werden und in die Spulen des passiven Transpondersystems eingekoppelt werden. Resonanzfrequenzen können beispielsweise in einem Bereich von 1 bis 100 MHz liegen, wobei die Frequenzanteile des anliegenden Druckes bei beispielsweise zwischen 0,1 und 50 Hz liegen können. Bei einer Verwendung an einem Blutgefäß kann die Hauptkomponente beispielsweise ein mit ca. 1 Hz sich periodisch wiederholender Herzschlag sein.

Die Signalquelle kann dann ein Signal mit den beiden interessierenden Frequenzen ωₐ und ω_{b} abgeben und an den Ausgang des Richtkopplers senden. Der Richtkoppler kann das Signal an seinen Eingang übertragen und an die Auslesespule weitergeben. Eine Direktübertragung des Signals von der Signalquelle an den Mischer erfolgt aufgrund des Richtkopplers nicht (oder für reale Richtkoppler nur in sehr geringem Maße, mit einem Isolationsparameter von größer als 25 dB). Was jedoch normalerweise von der Auslesespule zu den Mischern gelangen kann, sind von der Spule aufgrund der Abweichung von der Systemimpedanz (z. B. 50 Ohm) reflektierte Signale.

Ändern nun die Sensoren des passiven Transpondersystems ihre Resonanzfrequenzen ωₐ und ω_{b} aufgrund von Variationen des Druckes, der an den kapazitiven Drucksensoren anliegt, so kann dies im reflektierten Signal gemessen werden, da dadurch eine Änderung der Impedanz der Auslesespule und somit des reflektiven Signals bewirkt wird.

Eine Heruntermischung des Signals und Filterung mit einem Tiefpassfilter mit den zu beobachtenden Resonanzfrequenzen ωₐ und ω_{b} ist vorteilhaft, da für eine große Messbandbreite auch der Rauschpegel normalerweise große Werte annehmen kann und dann die Veränderung des reflektierten Signals bei weit entfernten und zum Beispiel im Körpergewebe eingebetteten Sensoren relativ schwach werden kann. Die so erhaltene Information liegt dann also direkt im Basisband vor.

Die Trennung in Amplitude und Phase der beiden Signale ist vorteilhaft, da diese dadurch addiert werden können, ohne dass sie sich bei bestimmten Winkeln auslöschen.

Der Mischer kann vorteilhafterweise das Messsignal mit dem Referenzsignal, das durch die Signalquelle erzeugt wird, multiplizieren und so eine Frequenzverschiebung des Spektrums des Messsignals um die Referenzfrequenz bewirken. Der im Messsignal enthaltene Frequenzanteil bei der Referenzfrequenz kann dadurch auf Null heruntergemischt werden. Die anschließende Tiefpassfilterung kann vorteilhafterweise weitere Mischprodukte entfernen und das Rauschen reduzieren.

Es wird außerdem ein Verfahren zum Herstellen eines passiven Transponders wie oben beschrieben angegeben. Dabei werden die erste und/oder die zweite Leiterschleifenstruktur wie folgt hergestellt. Zunächst wird auf eine Stange eine Vielzahl von Trägerelementen aufgereiht. Jedes dieser Trägerelemente hat eine zylinderförmige Außenoberfläche, in welcher eine Ausnehmung eingebracht ist. Diese Ausnehmung hat eine Oberfläche, die ihrerseits ein Ausschnitt einer Zylinderfläche ist. Vorteilhafterweise hat diese Zylinderfläche die gleiche Krümmung wie die Außenoberfläche des Trägerelementes. Außerdem steht vorteilhafterweise die Zylinderachse dieser Zylinderfläche der Ausnehmung senkrecht zur Zylinderachse der Außenoberfläche des Trägerelements. Es können auf diese Weise eine Vielzahl der Trägerelemente auf die Stange aufgereiht werden, wobei jeweils die Zylinderachsen der zylinderförmigen Außenoberfläche benachbarter Trägerelemente senkrecht zueinander stehen.

Es wird nun ein erster Draht als erste Leiterschleifenstruktur um eine erste Gruppe der Trägerelemente gewickelt. Dabei zeichnet sich die erste Gruppe der Trägerelemente dadurch aus, dass die Zylinderachsen ihrer Außenoberflächen alle parallel zueinander stehen.

Es wird darüber hinaus ein zweiter Draht als zweite Leiterschleifenstruktur um eine zweite Gruppe der Trägerelemente gewickelt, wobei diese zweite Gruppe wiederum jene der Trägerelemente enthält, deren Zylinderachsen parallel zueinander stehen, jedoch senkrecht zu den Zylinderachsen der Trägerelemente der ersten Gruppe. Auf diese Weise können die erste und die zweite Leiterschleifenstruktur mit zueinander senkrecht stehenden Windungsachsen gewickelt werden.

Im Anschluss an das Wickeln kann die Stange aus allen Trägerelementen herausgezogen werden, so dass diese frei beweglich werden und aus den Wicklungen herausgeschoben werden können. Es bleiben also nur die gewickelten Leiterschleifenstrukturen übrig. Vorteilhafterweise können der erste und der zweite Draht jeweils so gewickelt werden, dass sie zunächst ein Trägerelement der entsprechenden Gruppe zumindest einmal vollständig umlaufen und dann zum jeweils benachbarten Trägerelement der gleichen Gruppe weiterverlaufen. Dieses können sie wiederum vollständig umlaufen, um dann zum nächsten weiteren Trägerelement der gleichen Gruppe weiter zu verlaufen. Auf diese Weise können Leiterschleifenstrukturen mit einer Vielzahl von nebeneinander angeordneten Windungen erzeugt werden. Durch die Verwendung der Trägerelemente wird es insbesondere möglich, auch so zu wickeln, dass die ersten und die zweiten Wicklungen jeweils in zwei gegenüberliegenden Flächen verlaufen. Außerdem ist es ohne Weiteres möglich, den Draht der ersten Leiterschleifenstruktur durch die Windungen der zweiten Leiterschleifenstruktur zu führen und den Draht der zweiten Leiterschleifenstruktur durch die Windungen der ersten Leiterschleifenstruktur zu führen, so dass sich eine stabile geflochtene Struktur ergibt. Es sei angemerkt, dass die Zylinderachse der Außenoberflächen der Trägerelemente den Windungsachsen der um dieses Trägerelement gewundenen Windung entsprechen kann und dass die Längsachse der Stange der Hauptachse des passiven Transpondersystems entspricht.

Im Folgenden soll die Erfindung anhand einiger Figuren beispielhaft erläutert werden. Gleiche Bezugszeichen entsprechen dabei gleichen oder entsprechenden Merkmalen. Die in den Beispielen gezeigten Merkmale können auch unabhängig vom entsprechenden Beispiel realisiert werden und zwischen den Beispielen kombiniert werden.

Es zeigt
- Figur 1: eine Messanordnung zur Bestimmung einer Richtcharakteristik eines Transponders,
- Figur 2: eine Richtcharakteristik eines Transponders mit zwei Spulen,
- Figur 3: einen gedruckten Schaltkreis zur Herstellung eines Transpondersystems,
- Figur 4: eine Seitenansicht dieses Transpondersystems,
- Figur 5: eine axiale Ansicht dieses Transpondersystems,
- Figur 6: ein Beispiel eines erfindungsgemäßen Transpondersystems,
- Figur 7: eine perspektivische Ansicht des in Figur 6 gezeigten Transpondersystems,
- Figur 8: eine Detailansicht des in den Figuren 6 und 7 gezeigten Transpondersystems,
- Figur 9: eine axiale Ansicht des in den Figuren 6 bis 8 gezeigten erfindungsgemäßen Transpondersystems,
- Figur 10: ein Beispiel einer erfindungsgemäßen Druckwellenmessvorrichtung,
- Figur 11: eine beispielhafte Schaltung zweier auf die gleiche Frequenz abgestimmter Schwingkreise,
- Figur 12: die Resonanzverschiebung der in Figur 11 gezeigten Schwingkreise, und
- Figur 13: ein Beispiel eines Herstellungsverfahrens.

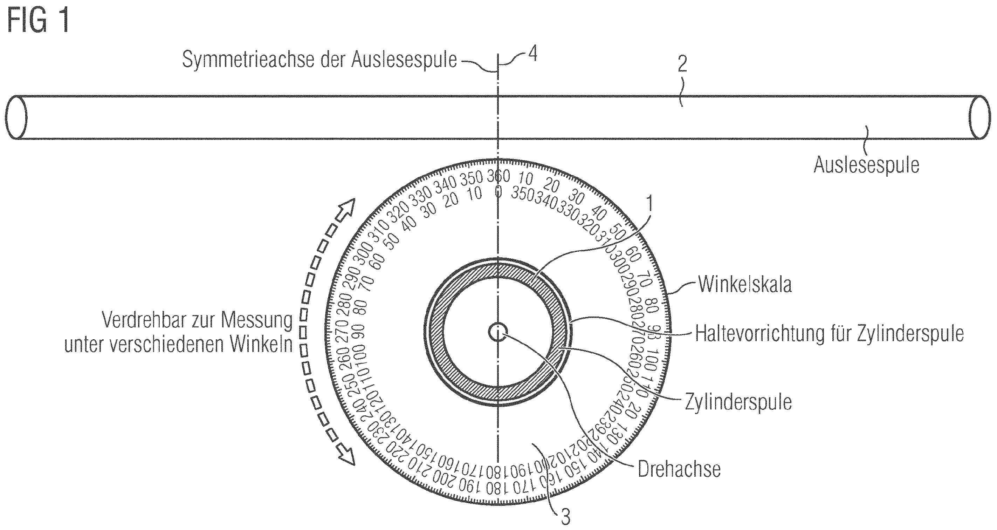

Figur 1 zeigt eine Messanordnung zur Vermessung der Richtcharakteristik eines Transponders 1. Der Transponder 1 ist hierbei gegenüber einer Auslesespule 2 mit variierbarer Orientierung angeordnet. Die Messvorrichtung weist eine optionale Winkelskala 3 auf, mit der die Orientierung des Transponders messbar ist. Die Auslesespule 2 ist hierbei eine Zylinderspule, die in Figur 1 von der Seite zu erkennen ist. Der Transponder 1 ist gegenüber der Auslesespule 2 so angeordnet, dass eine Symmetrieachse 4 der Auslesespule 2 eine Zylinderachse des Transponders 1 schneidet, wobei die Symmetrieachse 4 sowohl senkrecht auf der Auslesespule 2, als auch auf der Zylinderachse des Transponders 1 steht. Der Transponder 1 weist hier sattelförmige Spulen auf, die auf zylinderförmigen Oberflächen des Transponders 1 angeordnet sind und zwar dergestalt, dass die Spulenachsen senkrecht zur Längsachse des Transponders 1 stehen und die Symmetrieachse 4 schneiden oder zu dieser koaxial liegen. Die Spulenachsen schneiden außerdem die Zylinderachse des Transponders 1, hier die Drehachse.

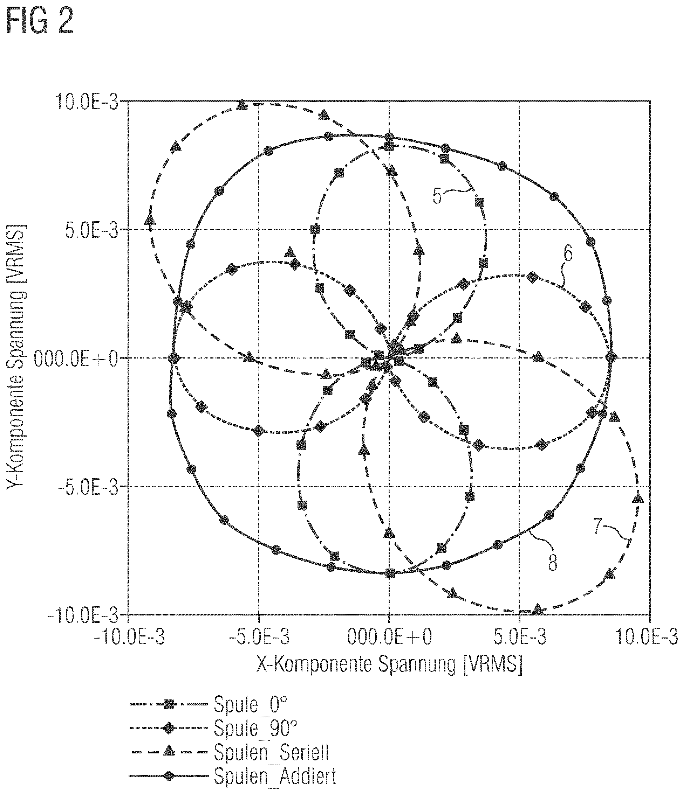

Figur 2 zeigt eine Richtcharakteristik, wie sie für einen Transponder mit der in Figur 1 gezeigten Messanordnung gemessen wird, wenn der Transponder 2 zylinderförmige Spulen aufweist, deren Spulenachsen senkrecht aufeinander stehen und einander schneiden. Figur 2 zeigt dabei die X-Komponente der in der Auslesespule induzierten Spannung und die Y-Komponente der in der Auslesespule induzierten Spannung, wobei die Spulenachse der einen Spule des Transponders in X-Richtung verläuft und die Spulenachse der anderen Spule des Transponders in Y-Richtung.

Die gepunktete Linie 5 (Quadrate) in Figur 2 zeigt die Richtcharakteristik jener Spule des Transponders, deren Spulenachse in Y-Richtung orientiert ist. Die klein gestrichelte Linie 6 (Punkte) zeigt die Richtcharakteristik jener Spule des Transponders, deren Spulenachse in X-Richtung orientiert ist. Zu erkennen ist, dass die beiden einzelnen Spulen des Transponders 1 eine ausgeprägt 8-förmige Richtcharakteristik haben, wobei die Richtcharakteristiken der beiden Spulen um 90° gegeneinander gedreht sind, da auch die Spulen um 90° gegeneinander gedreht sind. Schaltet man die beiden Spulen des Transponders 1 einfach in Reihe, so ergibt sich die groß gestrichelte Richtcharakteristik 7 (Dreiecke), die ebenfalls 8-förmig ist und gegenüber den Richtcharakteristiken der beiden Einzelspulen um 45° gedreht liegt. Die durchgezogene Linie (Kreise) zeigt die Richtcharakteristik eines Transponders entsprechend der vorliegenden Erfindung. Dadurch, dass die Signale der beiden einzelnen Spulen aufgrund deren unterschiedlicher Resonanzfrequenzen voneinander unterscheidbar sind, können die Signale der beiden Spulen konstruktiv miteinander addiert werden. Es ergibt sich dadurch die mit 8 gekennzeichnete nahezu kreisförmige Richtcharakteristik. Hier ist es nicht von Bedeutung, in welcher Richtung in der XY-Ebene ausgelesen wird. Es wird stets eine gute Kopplung zwischen dem Transponder und der Auslesespule erzielt.

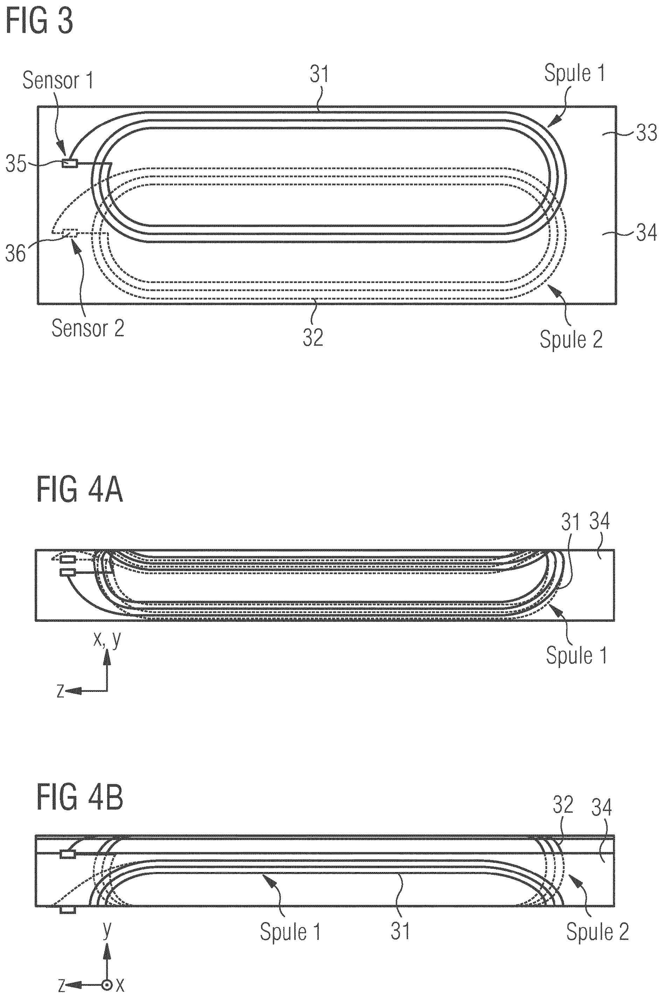

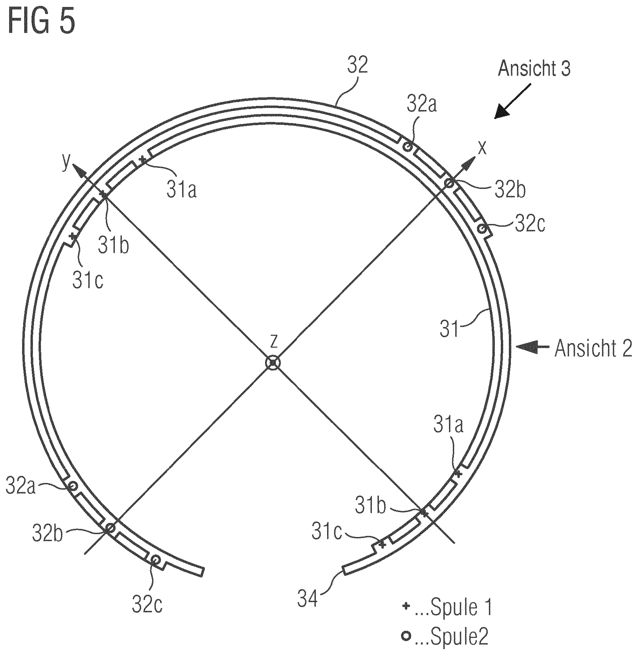

Die Figuren 3 bis 5 zeigen ein Beispiel eines nicht erfindungsgemäßen passiven Transponders. Dabei zeigt Figur 3 einen gedruckten Schaltkreis, aus dem der Transponder herstellbar ist, Figur 4 zeigt zwei Seitenansichten des so hergestellten Transponders und Figur 5 eine axiale Ansicht des so hergestellten Transponders.

Figur 3 zeigt eine gedruckte Schaltung 33 mit einer ersten Leiterschleifenstruktur 31 und einer zweiten Leiterschleifenstruktur. Dabei ist die Leiterschleifenstruktur 31 auf einer Vorderseite, in der Figur 3 dem Betrachter zugewandt, eines Substrates 34 aufgebracht und die zweite Leiterschleifenstruktur auf einer dem Betrachter abgewandten Rückseite des Substrats 34. Das Substrat 34 isoliert also die erste Leiterschleifenstruktur 31 und die zweite Leiterschleifenstruktur 32 elektrisch gegeneinander.

Die Leiterschleifenstrukturen 31 und 32 haben im gezeigten Beispiel jeweils drei Windungen. Jede der Leiterschleifenstrukturen 31 und 32 weist zwei gerade Bereiche auf, die zueinander parallel liegen und über zwei kreisförmig gebogene Bereiche miteinander verbunden sind. In den geraden Bereichen verlaufen die Leiterbahnen parallel zueinander und gerade und in den kreisförmig gebogenen Bereichen verlaufen die Leiterbahnen entlang einer Kreislinie und für alle Windungen der gleichen Leiterschleifenstruktur parallel zueinander. Mit der ersten Leiterschleifenstruktur 31 ist ein erster kapazitiver Drucksensor 35 gekoppelt. Der kapazitive Drucksensor 35 ist dabei zwischen den zwei Enden der Leiterschleifenstruktur 31 gekoppelt. Entsprechend weist die Leiterschleifenstruktur 32 einen zweiten kapazitiven Drucksensor 36 auf, der wiederum zwischen den beiden Enden der Leiterschleifenstruktur 32 angeordnet ist. Der erste kapazitive Drucksensor 35 bildet mit der ersten Leiterschleifenstruktur 31 einen ersten Schwingkreis mit einer ersten Resonanzfrequenz. Der zweite kapazitive Drucksensor 36 bildet mit der zweiten Leiterschleifenstruktur 32 einen zweiten Schwingkreis mit einer zweiten Resonanzfrequenz.

Die erste Leiterschleifenstruktur 31 ist um eine Windungsachse gewunden, wobei die Windungsachse hier den Mittelpunkt der Leiterbahnen der Leiterschleifenstruktur 31 durchstößt und senkrecht auf dem Substrat 34 steht. In entsprechender Weise werden die Leiterbahnen der zweiten Leiterschleifenstruktur 32 um eine zweite Spulenachse gewunden, die wiederum durch den Mittelpunkt der Leiterbahn der zweiten Leiterschleifenstruktur 32 verläuft und senkrecht auf dem Substrat 34 steht.

Aus der in Figur 3 gezeigten Struktur kann ein wie in Figur 4 gezeigter nicht erfindungsgemäßer passiver Transponder hergestellt werden, indem das Substrat 34 um eine Achse gebogen wird, die parallel zu den Längsseiten des Substrats 34 verläuft, die parallel liegen zu den geraden Abschnitten der Leiterschleifenstrukturen 31 und 32. Diese Richtung soll im Folgenden als Z-Richtung bezeichnet werden.

Figur 4A zeigt diese Ausgestaltung des Transponders in einer ersten Richtung, die senkrecht zur Z-Richtung und in einem Winkel von 45° zur X-Achse und zur Y-Achse steht. Die Teilfigur 4B zeigt den Transponder in Richtung der X-Achse betrachtet.

Die Leiterschleifenstrukturen 31 und 32 können vorteilhaft so bemessen sein, dass, wenn das Substrat 34 in der beschriebenen Weise gebogen wird, die geraden Bereiche der Leiterschleifenstrukturen einander gerade diametral bezüglich jener Achse, um die das Substrat 34 gebogen wurde, gegenüberliegen. Das Substrat 34 wird dabei vorzugsweise in eine Kreisform so gebogen, dass die Spulenachsen der ersten Leiterschleifenstruktur und der zweiten Leiterschleifenstruktur 32 im gewünschten Winkel zueinander stehen, vorzugsweise senkrecht zueinander.

Figur 5 zeigt die in der Figur 4 gezeigte Ausgestaltung des Transponders in der Blickrichtung in Richtung Z-Achse. Die in Figur 4A gezeigte Ansicht ergibt sich bei Betrachtung der in Figur 5 gezeigten Ansicht von rechts und die in Figur 4B gezeigte Ansicht ergibt sich bei Betrachtung von der rechten oberen Ecke aus.

Es ist zu erkennen, dass das Substrat zu einer Kreisform gebogen wurde. Dabei liegt die erste Leiterschleifenstruktur 31 auf einer inneren Oberfläche des so entstehenden zylinderförmigen Substrats und die zweite Leiterschleifenstruktur 32 auf seiner Außenoberfläche. Die Längsbereiche 31a, 31b und 31c der ersten Leiterschleifenstruktur 31 liegen einander bezüglich derer die geraden Abschnitte 31a, 31b, 31c der gleichen Windung der ersten Leiterschleifenstruktur 31 den gleichen Abstand haben. In entsprechender Weise liegen einander die geraden Abschnitte 32a, 32b, 32c bezüglich der YZ-Ebene gegenüber, bezüglich derer die geraden Abschnitte 32a, 32b, 32c der gleichen Windung den gleichen Abstand haben.

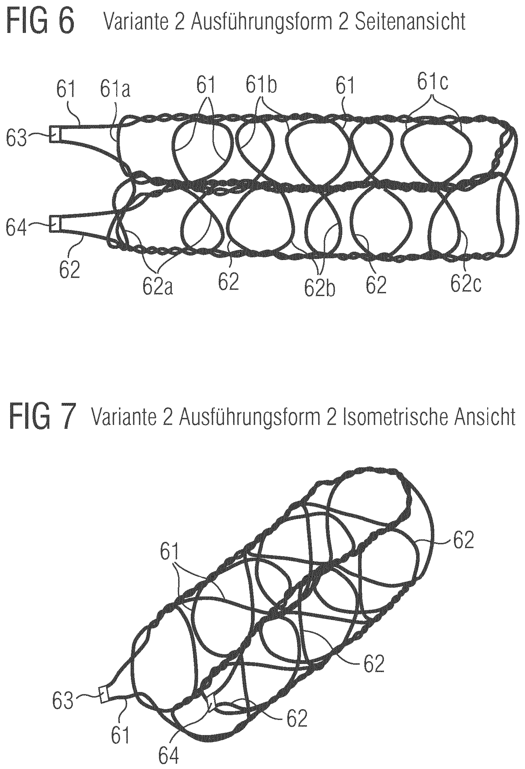

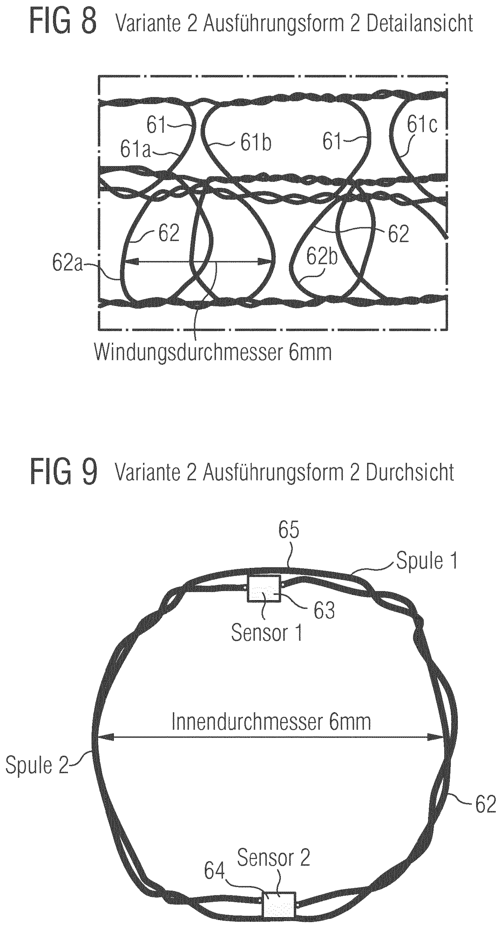

Die Figuren 6 bis 9 zeigen ein weiteres Beispiel eines erfindungsgemäßen passiven Transponders. Dabei zeigt Figur 6 eine Seitenansicht in Richtung senkrecht zur Z-Achse, die der Längsachse oder Hauptachse des Transponders entspricht, Figur 7 eine perspektivische Ansicht und Figur 8 eine Detailansicht. Figur 9 zeigt eine Ansicht in Richtung der Z-Achse.

In dieser Ausgestaltung der Erfindung ist ein erster Draht 61 zu einer ersten Leiterschleifenstruktur 61 gebogen und ein zweiter Draht 62 zu einer zweiten Leiterstruktur 62. Dabei weist die erste Leiterschleifenstruktur 61 eine Mehrzahl an in Richtung einer Hauptachse, die hier die Längsachse, also die Z-Richtung des Transponders ist, nebeneinander angeordneten Windungen 61a, 61b, 61c auf, wobei jede der Windungen der ersten Leiterschleifenstruktur 61 um eine eigene Windungsachse gewunden ist. Die ersten Windungsachsen stehen dabei parallel zueinander und sind voneinander um einen nicht verschwindenden Abstand beabstandet. Die gezeigte Ausgestaltung weist außerdem eine zweite Leiterschleifenstruktur 62 mit einer Mehrzahl in einer Richtung der Hauptachse nebeneinander angeordneten Windungen 62a, 62b, 62c auf, wobei wiederum jede der Windungen 62a, 62b, 62c der zweiten Leiterschleifenstruktur 62 um eine eigene zweite Windungsachse gewunden ist. Die zweiten Windungsachsen stehen wiederum parallel zueinander und sind voneinander um einen nicht verschwindenden Abstand beabstandet. Im gezeigten Beispiel stehen außerdem die Windungsachsen der ersten Windungen 61a, 61b, 61c senkrecht zu den Windungsachsen der zweiten Windungen 62a, 62b, 62c. Im gezeigten Beispiel weist außerdem die Leiterschleifenstruktur 61 auf jeder der Windungsachsen zwei Windungen auf, die einander bezüglich der Z-Achse gegenüber liegen. Darüber hinaus weist auch die zweite Leiterschleifenstruktur 62 für jede der Windungsachsen zwei einander bezüglich der Z-Achse gegenüberliegende Windungen auf.

Wie in Figur 8 im Detail zu erkennen ist, sind die Windungen 61a, 61b, 61c der ersten Leiterschleifenstruktur 61 mit den Windungen 62a, 62b der zweiten Leiterschleifenstruktur 62 verwoben. Es wird hierzu der Draht der ersten Leiterschleifenstruktur 61 zunächst zu einer im Wesentlichen kreisförmigen Windung gebogen, wobei die Windung dadurch verschlossen wird, dass der Draht um den Draht der zweiten Leiterschleifenstruktur 62 gewickelt wird, wo dieser zwischen zwei Windungen 62a und 62b der zweiten Leiterschleifenstruktur verläuft. Entsprechend wird der Draht der zweiten Leiterschleifenstruktur 62 zu einer Windung 62a gebogen, die dadurch geschlossen wird, dass der Draht um den Draht der ersten Leiterschleifenstruktur 61 gewickelt wird, wo dieser zwischen zwei benachbarten Windungen 61a und 61b der ersten Leiterschleifenstruktur verläuft. Auf diese Weise wird für jede der Leiterschleifenstrukturen 61 und 62 jeweils der Draht zu einer Windung gebogen, dann entlang eines geraden Bereichs parallel zur Zylinderachse des Transponders zur benachbarten Windung der gleichen Leiterschleifenstruktur geführt, dort wiederum in eine Windung gebogen und weiter in einem geraden Bereich zur benachbarten Windung der gleichen Leiterschleifenstruktur geführt, was für die Anzahl der Windungen in der gleichen Fläche der entsprechenden Leiterschleifenstruktur wiederholt wird. Die zweite Leiterschleifenstruktur wird entsprechend zu Windungen 62a, 62b gewunden, die wiederum über gerade Bereiche parallel zur Längsachse des Transponders geführt werden. Dabei werden die Windungen der zweiten Leiterschleifenstruktur 62 jeweils in einem Teilbereich der Windungen um den geraden Bereich der ersten Leiterschleifenstruktur 61 gewunden. Entsprechend werden die Windungen 61a, 61b, 61c der ersten Leiterschleifenstruktur 61 im Bereich der Windung um den geraden Bereich der angrenzenden zweiten Leiterschleifenstruktur gewunden.

Es sei darauf hingewiesen, dass die hier gezeigte Form der Wickelung lediglich ein vorteilhaftes Beispiel darstellt, dass eine gute mechanische Stabilität bewirkt. Es ist jedoch eine Vielzahl anderer Wicklungen denkbar, die zu einer gleichen Anordnung der Windungen der Leiterschleifenstrukturen 61 und 62 zueinander führt. Es ist auch möglich, dass die Leiter der Leiterschleifenstrukturen 61 und 62 auf andere Weise, beispielsweise durch Verkleben oder Zusammenbinden miteinander verbunden werden.

Figur 9 zeigt eine Ansicht des in den Figuren 6 bis 8 gezeigten Beispiels des erfindungsgemäßen Transponders in Richtung der Längsachse des Transponders, also in Richtung der Z-Achse, gesehen. Es ist zu erkennen, dass die Leiterschleifenstrukturen 61 und 62 einen ungefähr kreisförmigen Umfang beschreiben, so dass der gesamte Transponder ungefähr eine Zylinderform beschreibt.

Die erste Leiterschleifenstruktur 62 ist auch hier an ihren Enden mit einem ersten kapazitiven Drucksensor 63 gekoppelt und die zweite Leiterschleifenstruktur 62 mit einem zweiten kapazitiven Drucksensor 64.

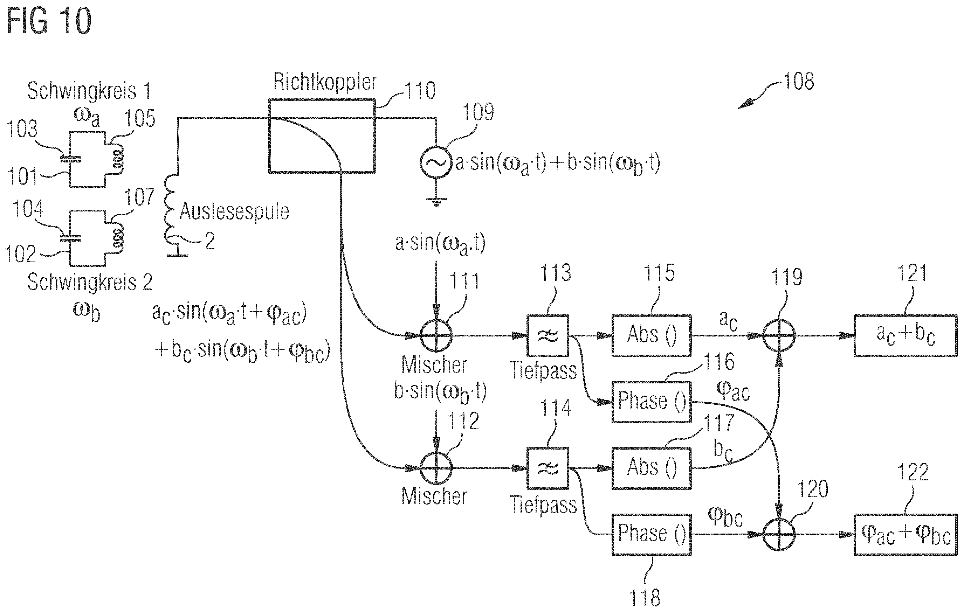

Figur 10 zeigt ein beispielhaftes Schaltbild einer erfindungsgemäßen Druckwellenmessvorrichtung entsprechend der Erfindung. Die erfindungsgemäße Druckwellenmessvorrichtung weist dabei einen passiven Transponder wie vorstehend beschrieben auf, der hier durch einen ersten Schwingkreis 101 und einen zweiten Schwingkreis 102 dargestellt wird. Der erste Schwingkreis 101 weist einen ersten kapazitiven Drucksensor 103 auf und der zweite Schwingkreis 2 weist einen zweiten kapazitiven Drucksensor 104 auf, die in Figur 10 als Kapazitäten dargestellt sind. Darüber hinaus weist der erste Schwingkreis 101 eine erste Leiterschleifenstruktur 105 auf und der zweite Schwingkreis eine zweite Leiterschleifenstruktur 107 auf.

Die in Figur 10 gezeigte Messvorrichtung weist außerdem eine Ausleseeinheit 108 auf, mit der die Schwingkreise 101 und 102 auslesbar sind. Die Ausleseeinheit 108 weist eine Auslesespule 2 auf. Mittels einer Signalquelle 109 ist ein Signal, also ein Stromsignal, mit zwei überlagerten Frequenzen ωₐ und ω_{b} erzeugbar, wobei der Anteil mit der Frequenz ωₐ mit einer Amplitude a erzeugt wird und der Anteil mit der Frequenz ω_{b} mit einer Amplitude b. Es ergibt sich also die Signalform a · sin(ωₐt) + b · sin(ω_{b}t). Das so erzeugte Signal wird über einen Richtkoppler 110 in die Auslesespule 2 eingespeist. Der Richtkoppler ist mit einem ersten Mischer 111 und einem zweiten Mischer 112 gekoppelt, in die ein vom Richtkoppler 110 von der Auslesespule 2 empfangenes Signal einspeisbar ist. Im ersten Mischer 111 wird das von der Auslesespule erhaltene Signal mit dem Referenzsignal a · sin(ωₐt) gemischt und im zweiten Mischer 112 mit dem Referenzsignal b · sin(ω_{b}t).

Das vom ersten Mischer 111 ausgegebene Signal wird dann einem Tiefpassfilter 113 zugeführt. Das vom zweiten Mischer 112 ausgegebene Signal wird einem Tiefpassfilter 114 zugeführt. Der erste Tiefpassfilter 113 wird dann von der Ausleseeinheit 108 in einen Absolutbetrag bzw. eine Amplitude 115 und eine Phase 116 aufgeteilt. Das vom zweiten Tiefpassfilter 114 ausgegebene Signal wird ebenfalls in eine Amplitude 117 und eine Phase 118 aufgeteilt. Mittels einer Addition 119 werden die Absolutwerte 115 und 117 des ersten und zweiten Signals addiert, um eine Summe 121 zu erhalten. Die Phasen 116 und 118 des ersten und des zweiten Signals werden mit einem weiteren Addierer 120 addiert, um eine Summe 122 zu erhalten. Das auf diese Weise erhaltene Signal 121 und 122 weist dann eine kreisförmige Richtcharakteristik auf, wie diese in Figur 2 mit der durchgezogenen Linie (Kreise) gezeigt ist.

Es sei darauf hingewiesen, dass die Mischer 111, 112 und die Tiefpassfilter 113, 114 der Verbesserung des Signals dienen. Sie sind daher optional.

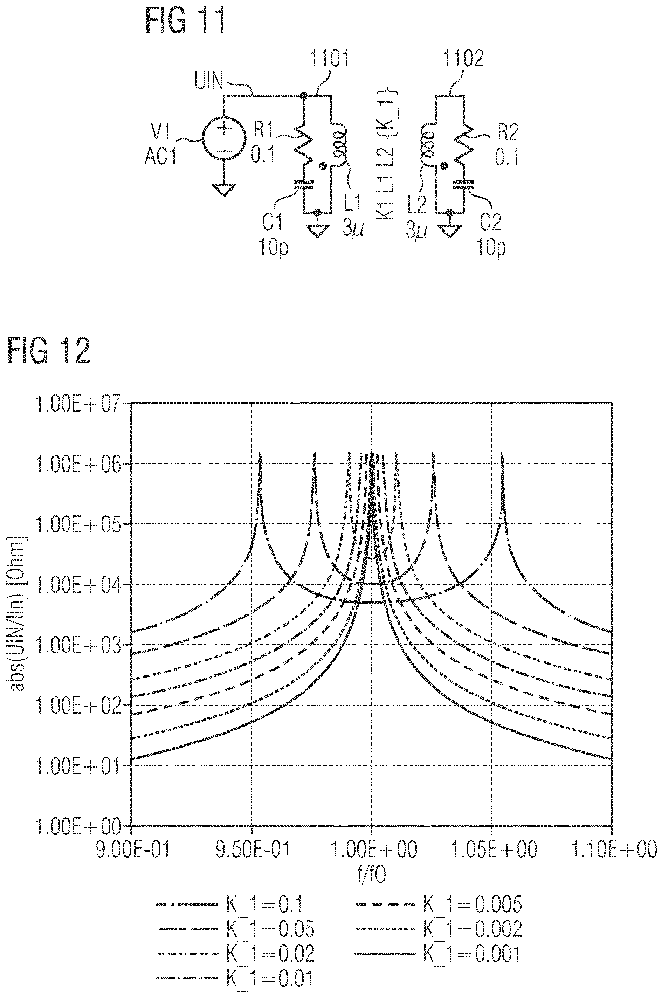

Figur 11 zeigt eine Schaltung, in der zwei auf eine gleiche Frequenz abgestellte Schwingkreise 1101 und 1102 über zwei Spulen L1 und L2 mit einem Kopplungsfaktor K1 miteinander verkoppelt sind. Deren Wechselwirkung wird in Abhängigkeit von K1 dargestellt. Je größer K1 ist, desto größer ist die Wechselwirkung zwischen den Spulen L1 und L2 und desto größer ist eine sich ergebende Verstimmung. Der erste Schwingkreis weist neben der Spule L1, die im gezeigten Beispiel eine Induktivität von 3 µH hat, eine Kapazität C1 von 10 pF und einen Widerstand R1 mit einem Wert von 0,1 Ω auf. Der zweite Schwingkreis 1102 weist die zweite Spule L2, eine zweite Kapazität C2 und einen zweiten Widerstand R2 mit den gleichen Werten wie im ersten Schwingkreis 1101 auf, so dass die beiden Schwingkreise 1101 und 1102 die gleiche Resonanzfrequenz haben. An den ersten Schwingkreis 1101 wird eine Spannung V1 angelegt, mit der dieser zum Schwingen anregbar ist.

Figur 12 zeigt die Impedanz UIN/IIn, die im ersten Schwingkreis gemessen wird, abhängig von der Frequenz f/f₀ für verschiedene Kopplungsfaktoren K1. Der Wert f₀ ist dabei die Resonanzfrequenz der beiden Schwingkreise 1101 und 1102 in ungekoppeltem Zustand. Die verschiedenen Kurven zeigen dabei unterschiedliche Werte des Kopplungsfaktors K1. Es ist zu erkennen, dass mit einer sehr kleinen Kopplung von K1 = 0,001 (durchgezogene Linie) sich die Resonanzfrequenz nicht verschiebt. Wird jedoch die Kopplung erhöht, so ist zu erkennen, dass die Resonanzfrequenzen der beiden Schwingkreise auseinanderlaufen.

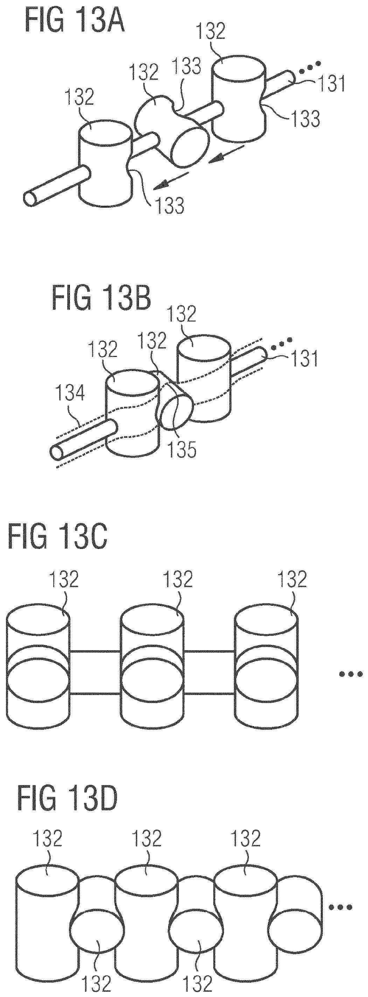

Figur 13 zeigt ein bildhaftes Herstellungsverfahren zur Herstellung eines erfindungsgemäßen passiven Transponders. Es werden dabei in einem ersten Schritt eine Vielzahl von Trägerelementen 132 auf eine Stange 131 aufgereiht. Jedes Trägerelement 132 weist dabei eine zylinderförmige Außenoberfläche auf, in die eine zylinderteilflächenförmige Ausnehmung 133 eingebracht ist. Die Trägerelemente 132 werden auf der Stange so aufgereiht, dass die Zylinderachsen ihre zylinderförmigen Außenoberflächen zu einer Längsrichtung der Stange 131 senkrecht stehen, und dass außerdem jeweils eines der Trägerelemente 132 in der zylinderteilflächenförmigen Ausnehmung 133 eines benachbarten der Trägerelemente 132 liegt. Dieser Zustand ist in Figur 13B zu erkennen.

Trägerstrukturen können auch, wie in den Figuren 13C und 13D gezeigt hergestellt werden, wobei die in 13D hergestellte Variante sich aus dem in den Teilfiguren A und B gezeigten Verfahren ergeben kann.

Um die Trägerelemente 132 wird dann, wie in Figur 13B gepunktet angedeutet, ein erster Draht als erste Leiterschleifenstruktur und ein zweiter Draht 135 als zweite Leiterschleifenstruktur 135 gewickelt. Die Wicklung kann dabei so erfolgen, dass sich beispielsweise eine Struktur wie in den Figuren 6 bis 9 gezeigt ergibt. Nach Abschluss des Wickelns können die Elemente 132 entfernt werden, so dass nur noch die Leiterschleifenstrukturen zurückbleiben. Hierzu kann in den Figuren 13A und 13B beispielsweise die Stange 131 entfernt werden. Vorzugsweise werden die Drähte 134 und 135 jeweils zumindest einmal um jedes der Trägerelemente 132 herumgeführt, so dass sich jeweils eine Windung ergibt.

## Patentansprüche

1. Passives Transpondersystem aufweisend
eine erste Leiterschleifenstruktur (61), wobei die erste Leiterschleifenstruktur (61) eine Mehrzahl an in Richtung einer Hauptachse des Transpondersystems nebeneinander angeordneten Gruppen von Windungen mit jeweils zumindest einer Windung (61a, 61b, 61c) aufweist, wobei jede der Windungen der gleichen Gruppe der ersten Leiterschleifenstruktur (61) um eine gemeinsame erste Windungsachse gewunden ist, wobei die ersten Windungsachsen unterschiedlicher Gruppen voneinander jeweils um einen nicht verschwindenden Abstand beabstandet sind,
eine zweite Leiterschleifenstruktur (62), wobei die zweite Leiterschleifenstruktur (62) eine Mehrzahl an in Richtung der Hauptachse nebeneinander angeordneten Gruppen von Windungen mit jeweils zumindest einer Windung (62a, 62b, 62c) aufweist, wobei jede der Windungen der gleichen Gruppe der zweiten Leiterschleifenstruktur (62) um gemeinsame zweite Windungsachse gewunden ist, wobei die zweiten Windungsachsen unterschiedlicher Gruppen voneinander um einen nicht verschwindenden Abstand beabstandet sind, einen ersten kapazitiven Drucksensor (63),
einen zweiten kapazitiven Drucksensor (64),
wobei der erste kapazitive Drucksensor (63) mit der ersten Leiterschleifenstruktur (61) zu einem ersten Schwingkreis mit einer ersten Resonanzfrequenz elektrisch gekoppelt ist,
wobei der zweite kapazitive Drucksensor (64) mit der zweiten Leiterschleifenstruktur (62) zu einem zweiten Schwingkreis mit einer zweiten Resonanzfrequenz elektrisch gekoppelt ist,
wobei die ersten Windungsachsen und die zweiten Windungsachsen in einem nicht verschwindenden Winkel zueinander stehen, wobei der nicht verschwindende Winkel größer als Null und kleiner als 180° ist.

2. Passives Transpondersystem nach dem vorhergehenden Anspruch,
wobei die erste Resonanzfrequenz und die zweite Resonanzfrequenz sich zumindest so weit unterscheiden, dass sie sich nicht schwebend überlagern.

3. Passives Transpondersystem nach einem der vorhergehenden Ansprüche, wobei die erste Leiterschleifenstruktur (61) und die zweite Leiterschleifenstruktur (62) als Flachspulen ausgebildet sind, die auf Oberflächen einer Trägerstruktur (34) angeordnet sind.

4. Passives Transpondersystem nach dem vorhergehenden Anspruch,
wobei die Trägerstruktur (34) teilzylinderförmig oder zylinderförmig oder teilschlauchförmig oder teilrohrförmig oder schlauchförmig oder rohrförmig ist, wobei vorzugsweise die Trägerstruktur (34) ein Kunststoffrohr aufweist oder ist.

5. Passives Transpondersystem nach einem der beiden vorhergehenden Ansprüche 3 oder 4, wobei die erste Leiterschleifenstruktur (61) auf einer innenseitigen Oberfläche der Trägerstruktur (34) angeordnet ist und die zweite Leiterschleifenstruktur (62) auf einer außenseitigen Oberfläche der Trägerstruktur (34) angeordnet ist.

6. Passives Transpondersystem nach einem der vorhergehenden Ansprüche, wobei die ersten Windungsachsen und/oder die zweiten Windungsachsen zueinander parallel stehen.

7. Passives Transpondersystem nach dem vorhergehenden Anspruch,
wobei die Windungen (61a, 61b, 61c) der ersten Leiterschleifenstruktur (61) sich in zwei bezüglich der Hauptachse gegenüberliegenden Flächen erstrecken auf denen die ersten Windungsachsen senkrecht stehen, und
wobei die Windungen (62a, 62b, 62c) der zweiten Leiterschleifenstruktur (62) sich in zwei bezüglich der Hauptachse gegenüberliegenden Flächen erstrecken, auf denen die zweiten Windungsachsen senkrecht stehen, wobei vorzugsweise die ersten Windungsachsen in Richtung entlang der Hauptachse zwischen den zweiten Windungsachsen liegen.

8. Passives Transpondersystem nach Anspruch 7, wobei die kapazitiven Drucksensoren (63, 64) mit ihren Druck-messenden Flächen jeweils in einer der gegenüberliegenden Flächen liegen, in denen sich die Leiterschleifenstruktur (61, 62) des entsprechenden Schwingkreises erstreckt.

9. Passives Transpondersystem nach einem der vorhergehenden Ansprüche,
wobei die erste und die zweite Leiterschleifenstruktur (61, 62) eine Gefäßstütze für ein Blutgefäß oder einen Stent bilden.

10. Druckwellenmessvorrichtung, aufweisend
ein passives Transpondersystem nach einem der vorhergehenden Ansprüche, sowie eine Ausleseeinheit (108),
wobei die Ausleseeinheit (108) eine Auslesespule (2) aufweist, die so gegenüber dem passiven Transpondersystem anordenbar ist, dass ein durch sie erzeugtes Magnetfeld zumindest eine der Leiterschleifenstrukturen (61, 62) des Transpondersystems durchsetzt,
weiter aufweisend eine Auswerteelektronik, mit der die Auslesespule (2) mit einem die Schwingkreise anregenden Signal beaufschlagbar ist und ein von der Auslesespule empfangenes Signal auswertbar ist.

11. Druckwellenmessvorrichtung nach dem vorhergehenden Anspruch, aufweisend eine Signalquelle (109), mit der ein Signal mit den Resonanzfrequenzen der Schwingkreise erzeugbar ist, sowie weiter aufweisend einen Richtkoppler (110), an dessen Ausgang die Signalquelle (109) elektrisch gekoppelt ist und an dessen Eingang die Auslesespule (2) gekoppelt ist.

12. Druckwellenmessvorrichtung nach einem der beiden vorhergehenden Ansprüche,
weiter aufweisend einen ersten Mischer (111) und einen hinter diesem angeordneten ersten Tiefpassfilter (113), wobei mit dem ersten Mischer (111) ein durch die Auslesespule von dem Transpondersystem empfangenes Signal der ersten Resonanzfrequenz heruntermischbar ist, und weiter aufweisend
einen weiteren Mischer (112) und einen hinter diesem angeordneten zweiten Tiefpassfilter (114), wobei mit dem weiteren Mischer (112) ein durch die Auslesespule (2) von dem Transpondersystem empfangenes Signal der zweiten Resonanzfrequenz heruntermischbar ist.

13. Druckwellenmessvorrichtung nach einem der Ansprüche 10 bis 12,
wobei die Druckwellenmessvorrichtung eingerichtet ist, das vom ersten Tiefpassfilter (113) ausgegebene Signal mit dem vom zweiten Tiefpassfilter (114) ausgegebenen Signal konstruktiv zu addieren.

14. Druckwellenmessvorrichtung nach einem der Ansprüche 10 bis 13,
wobei die Druckwellenmessvorrichtung eingerichtet ist, das vom ersten Tiefpassfilter (113) erzeugte Signal in Amplitude und Phase zu trennen, und das vom zweiten Tiefpassfilter (114) ausgegebene Signal in Amplitude und Phase zu trennen,
und weiter eingerichtet ist, die aus dem vom ersten Tiefpassfilter (113) erzeugten Signal getrennte Amplitude mit der aus dem vom zweiten Tiefpassfilter (114) erzeugten Signal getrennten Amplitude zu addieren, und vorzugsweise außerdem eingerichtet ist,
die aus dem vom ersten Tiefpassfilter (113) erzeugten Signal getrennte Phase mit der aus dem vom zweiten Tiefpassfilter (114) erzeugten Signal getrennten Phase zu addieren.

## Claims

1. Passive transponder system comprising
a first conductor loop structure (61), wherein the first conductor loop structure (61) has a plurality of groups of turns, the groups arranged next to one another in the direction of a main axis of the transponder system, each group of turns comprising at least one turn (61a, 61b, 61c), wherein each of the turns of the same group of the first conductor loop structure (61) is wound around a common first turn axis, wherein the first turn axes of different groups are in each case spaced apart from one another by a non-vanishing distance,
a second conductor loop structure (62), wherein the second conductor loop structure (62) has a plurality of groups of turns, the groups arranged next to one another in the direction of the main axis, each group comprising at least one turn (62a, 62b, 62c), wherein each of the turns of the same group of the second conductor loop structure (62) is wound around a common second turn axis, wherein the second turn axes of different groups are spaced apart from one another by a non-vanishing distance,
a first capacitive pressure sensor (63),
a second capacitive pressure sensor (64),
wherein the first capacitive pressure sensor (63) is electrically coupled to the first conductor loop structure (61) to form a first resonant circuit with a first resonant frequency,
wherein the second capacitive pressure sensor (64) is electrically coupled to the second conductor loop structure (62) to form a second resonant circuit with a second resonant frequency,
wherein the first turn axes and the second turn axes are at a non-vanishing angle to one another, wherein the non-vanishing angle is greater than zero and less than 180°.

2. Passive transponder system according to the preceding claim, wherein the first resonant frequency and the second resonant frequency differ from one another at least to such an extent that they do not superpose one another in such a way as to produce a beat.

3. Passive transponder system according to one of the preceding claims, wherein the first conductor loop structure (61) and the second conductor loop structure (62) are designed as flat coils which are arranged on surfaces of a carrier structure (34).

4. Passive transponder system according to the preceding claim, wherein the carrier structure (34) is shaped as part of a cylinder or as a cylinder or as part of a hose or as part of a tube or as a hose or as a tube, wherein preferably the carrier structure (34) has or is a plastic tube.

5. Passive transponder system according to one of the two preceding claims 3 or 4, wherein the first conductor loop structure (61) is arranged on an inner surface of the carrier structure (34) and the second conductor loop structure (62) is arranged on an outer surface of the carrier structure (34).

6. Passive transponder system according to one of the preceding claims, wherein the first turn axes and/or the second turn axes are parallel to one another.

7. Passive transponder system according to the preceding claim, wherein the turns (61a, 61b, 61c) of the first conductor loop structure (61) extend in two surfaces located opposite one another in relation to the main axis, the first turn axes being perpendicular to said surfaces, and
wherein the turns (62a, 62b, 62c) of the second conductor loop structure (62) extend in two surfaces located opposite one another in relation to the main axis, the second turn axes being perpendicular to said surfaces, wherein preferably the first turn axes are located between the second turn axes in the direction along the main axis.

8. Passive transponder system according to claim 7, wherein the capacitive pressure sensors (63, 64) are located with their pressure-measuring surfaces in each case in one of the opposing surfaces in which the conductor loop (61, 62) structure of the corresponding resonant circuit extends.

9. Passive transponder system according to one of the preceding claims,
wherein the first and the second conductor loop structure (61, 62) form a vessel support for a blood vessel or a stent.

10. Pressure wave measuring device comprising
a passive transponder system according to one of the preceding claims, and a reader unit (108),
wherein the reader unit (108) has a reader coil (2) which can be arranged relative to the passive transponder system in such a way that a magnetic field generated by said reader coil passes through at least one of the conductor loop structures (61, 62) of the transponder system,
further comprising evaluation electronics, by means of which a signal that excites the resonant circuits can be applied to the reader coil (2) and a signal received from the reader coil can be evaluated.

11. Pressure wave measuring device according to the preceding claim, comprising a signal source (109), by means of which a signal can be generated with the resonant frequencies of the resonant circuits, and further comprising a directional coupler (110), to the output of which the signal source (109) is electrically coupled and to the input of which the reader coil (2) is coupled.

12. Pressure wave measuring device according to one of the two preceding claims,
further comprising a first mixer (111) and a first low-pass filter (113) arranged behind it, wherein a signal with the first resonant frequency that is received by the reader coil from the transponder system can be downmixed by means of the first mixer (111), and further comprising
a further mixer (112) and a second low-pass filter (114) arranged behind it, wherein a signal with the second resonant frequency that is received by the reader coil (2) from the transponder system can be downmixed by means of the further mixer (112).

13. Pressure wave measuring device according to one of claims 10 to 12, wherein the pressure wave measuring device is designed to constructively add the signal output from the first low-pass filter (113) to the signal output from the second low-pass filter (114).

14. Pressure wave measuring device according to one of claims 10 to 13, wherein the pressure wave measuring device is configured to separate the signal generated by the first low-pass filter (113) into amplitude and phase and to separate the signal output from the second low-pass filter (114) into amplitude and phase,
and is further configured to add the amplitude separated from the signal generated by the first low-pass filter (113) to the amplitude separated from the signal generated by the second low-pass filter (114), and preferably is additionally configured to
add the phase separated from the signal generated by the first low-pass filter (113) to the phase separated from the signal generated by the second low-pass filter (114).

## Revendications

1. Système de transpondeur passif présentant
une première structure de boucle conductrice (61), dans lequel ladite première structure de boucle conductrice (61) présente une pluralité de groupes de spires disposés les uns à côté des autres dans la direction d'un axe principal du système de transpondeur avec respectivement au moins une spire (61a, 61b, 61c), dans lequel chacune des spires du même groupe de la première structure de boucle conductrice (61) est enroulée autour d'un premier axe d'enroulement commun, dans lequel les premiers axes d'enroulement de groupes différents sont espacés les uns des autres respectivement d'une distance différente de zéro,
une deuxième structure de boucle conductrice (62), dans lequel la deuxième structure de boucle conductrice (62) présente une pluralité de groupes de spires disposés les uns à côté des autres dans la direction de l'axe principal avec respectivement au moins une spire (62a, 62b, 62c), dans lequel chacune des spires du même groupe de la deuxième structure de boucle conductrice (62) est enroulée autour d'un deuxième axe de spire commun, dans lequel les deuxièmes axes de spire de groupes différents sont espacés les uns des autres d'une distance différente de zéro,
un premier capteur de pression capacitif (63),
un deuxième capteur de pression capacitif (64),
dans lequel le premier capteur de pression capacitif (63) est couplé électriquement à la première structure de boucle conductrice (61) en un premier circuit oscillant avec une première fréquence de résonance,
dans lequel le deuxième capteur de pression capacitif (64) est couplé électriquement à la deuxième structure de boucle conductrice (62) en un deuxième circuit oscillant avec une deuxième fréquence de résonance,
dans lequel les premiers axes d'enroulement et les deuxièmes axes d'enroulement forment un angle différent de zéro, dans lequel l'angle différent de zéro est supérieur à zéro et inférieur à 180°.

2. Système de transpondeur passif selon la revendication précédente, dans lequel la première fréquence de résonance et la deuxième fréquence de résonance diffèrent au moins suffisamment pour ne pas se superposer de manière flottante.

3. Système de transpondeur passif selon l'une quelconque des revendications précédentes, dans lequel la première structure de boucle conductrice (61) et la deuxième structure de boucle conductrice (62) sont réalisées comme des bobines plates qui sont disposées sur les surfaces d'une structure de support (34).

4. Système de transpondeur passif selon la revendication précédente, dans lequel la structure de support (34) est en forme de cylindre partiel ou en forme de cylindre ou en forme de tuyau partiel ou en forme de tube partiel ou en forme de tuyau ou en forme de tube, dans lequel de préférence la structure de support (34) présente ou est un tube en plastique.

5. Système de transpondeur passif selon l'une des deux revendications 3 ou 4 précédentes, dans lequel la première structure de boucle conductrice (61) est disposée sur une surface intérieure de la structure de support (34) et la deuxième structure de boucle conductrice (62) est disposée sur une surface extérieure de la structure de support (34).

6. Système de transpondeur passif selon l'une quelconque des revendications précédentes, dans lequel les premiers axes de spires et/ou les deuxièmes axes de spires sont parallèles entre eux.

7. Système de transpondeur passif selon la revendication précédente,
dans lequel les spires (61a, 61b, 61c) de la première structure de boucle conductrice (61) s'étendent dans deux surfaces opposées par rapport à l'axe principal auxquelles les premiers axes de spires sont perpendiculaires, et
dans lequel les spires (62a, 62b, 62c) de la deuxième structure de boucle conductrice (62) s'étendent dans deux surfaces opposées par rapport à l'axe principal, auxquelles les deuxièmes axes de spires sont perpendiculaires, dans lequel de préférence les premiers axes de spires se situent entre les deuxièmes axes de spires dans la direction le long de l'axe principal.

8. Système de transpondeur passif selon la revendication 7, dans lequel les capteurs de pression capacitifs (63, 64) se situent avec leurs surfaces de mesure de pression respectivement dans l'une des surfaces opposées dans lesquelles s'étend la structure de boucle conductrice (61, 62) du circuit oscillant correspondant.

9. Système de transpondeur passif selon l'une quelconque des revendications précédentes,
dans lequel la première et la deuxième structure de boucle conductrice (61, 62) forment un tuteur vasculaire pour un vaisseau sanguin ou un stent.

10. Dispositif de mesure d'ondes de pression, présentant
un système de transpondeur passif selon l'une quelconque des revendications précédentes, ainsi qu'une unité de lecture (108),
dans lequel l'unité de lecture (108) présente une bobine de lecture (2) qui peut être disposée en face du système de transpondeur passif, de telle sorte qu'un champ magnétique généré par celle-ci traverse au moins l'une des structures de boucle conductrice (61, 62) du système de transpondeur,
présentant en outre une électronique d'évaluation avec laquelle la bobine de lecture (2) peut être alimentée avec un signal excitant les circuits oscillants et un signal reçu par la bobine de lecture peut être évalué.

11. Dispositif de mesure d'ondes de pression selon la revendication précédente, présentant une source de signal (109) avec laquelle un signal peut être généré avec les fréquences de résonance des circuits oscillants, et présentant en outre un coupleur directionnel (110) à la sortie duquel la source de signal (109) est couplée électriquement et à l'entrée duquel la bobine de lecture (2) est couplée.

12. Dispositif de mesure d'ondes de pression selon l'une des deux revendications précédentes,
présentant en outre un premier mélangeur (111) et un premier filtre passe-bas (113) disposé derrière celui-ci, dans lequel un signal de la première fréquence de résonance reçu par la bobine de lecture du système de transpondeur peut être abaissé par mélange avec le premier mélangeur (111), et présentant en outre
un autre mélangeur (112) et un deuxième filtre passe-bas (114) disposé derrière celui-ci, dans lequel un signal de la deuxième fréquence de résonance reçu par la bobine de lecture (2) du système de transpondeur peut être abaissé par mélange avec l'autre mélangeur (112).

13. Dispositif de mesure d'ondes de pression selon l'une quelconque des revendications 10 à 12, dans lequel le dispositif de mesure d'ondes de pression est conçu pour additionner par construction le signal émis par le premier filtre passe-bas (113) au signal émis par le deuxième filtre passe-bas (114).

14. Dispositif de mesure d'ondes de pression selon l'une quelconque des revendications 10 à 13, dans lequel le dispositif de mesure d'ondes de pression est conçu pour séparer en amplitude et en phase le signal généré par le premier filtre passe-bas (113), et pour séparer en amplitude et en phase le signal émis par le deuxième filtre passe-bas (114),
et est en outre conçu pour additionner l'amplitude séparée du signal généré par le premier filtre passe-bas (113) à l'amplitude séparée du signal généré par le deuxième filtre passe-bas (114), et est de préférence en outre conçu
pour additionner la phase séparée du signal généré par le premier filtre passe-bas (113) à la phase séparée du signal généré par le deuxième filtre passe-bas (114).
